Europäisches Patentamt

(19) European Patent Office

Office européen des brevets

(11) Publication number: **0 391 971 B1**

# EUROPEAN PATENT SPECIFICATION

(45) Date of publication of patent specification: **16.03.94**  (51) Int. Cl.⁵: **A61K 31/16**

(21) Application number: **89901233.0**

(22) Date of filing: **15.12.88**

(86) International application number:
**PCT/US88/04445**

(87) International publication number:
**WO 89/05637 (29.06.89 89/14)**

(54) **IMPROVING TOXICITY PROFILES IN CHEMOTHERAPY.**

(30) Priority: **22.12.87 US 136721**

(43) Date of publication of application:
**17.10.90 Bulletin 90/42**

(45) Publication of the grant of the patent:
**16.03.94 Bulletin 94/11**

(84) Designated Contracting States:
**AT BE CH DE FR GB IT LI LU NL SE**

(56) References cited:
**US-A- 3 892 824**
**US-A- 4 314 989**
**US-A- 4 424 216**
**US-A- 4 676 979**

**WALTER REED ARMY INSTITUTE OF RE-
SEARCH, September 1979, US; T.R.
SWEENEY, p. 12&NUM;**

**CANCER CLINICAL TRIALS, vol. 4, 1981, US;
WASSERMANN et al., pp. 3-6&NUM;**

(73) Proprietor: **U.S. BIOSCIENCE**
**920-B Harvest Drive**
**P.O. Box 220**
**Blue Bell, PA 19422(US)**

(72) Inventor: **SCHEIN, Philip**
**605 Old Gulph Road**
**Bryn Mawr, PA 19010(US)**
Inventor: **PIPER, James, R.**
**3128 Dolly Ridge Dr.**
**Birmingham, AL 35243(US)**

(74) Representative: **von Füner, Alexander, Dr. et al**
**Patentanwälte v. Füner, Ebbinghaus, Finck**
**Mariahilfplatz 2 & 3**
**D-81541 München (DE)**

INTERNATL. JOURNAL RADIATION, ONCOL-
OGY, BIOL., PHYS., vol. 10, no. 9, September
1984, US; VALERIOTE et al., pp.
1561-1564&NUM;

CHEMICAL ABSTRACTS, vol. 106, no. 9, 02
March 1987, Columbus, OH (US); FURUKAWA
et al., p. 49, no. 61084j&NUM;

PROCEEDINGS ANNUAL MEET-
INGS/AMERICAN ASSOCIATION OF CANCER
RESEARCH, vol. 31, 23-26 March 1990; D.
GREEN et al., no. A1957&NUM;

PROCEEDINGS ANNUAL MEET-
INGS/AMERICAN ASSOCIATION OF CANCER
RESEARCH, vol. 30, 24-27 May 1989; D.C.
GREEN et al., no. A1871&NUM;

**Description**

Cancer chemotherapy has been practiced for many years with many different therapeutic agents. A major drawback of this therapy scheme is the toxicity of the chemotherapeutic agents. Agents capable of destroying invading cancer cells are unfortunately often quite toxic to normal cells. Thus, employers of and recipients of chemotherapeutic techniques have a great need for either non-toxic (to normal cells) therapeutic agents or additional agents capable of decreasing the toxicity of chemotherapeutic agents. The present invention is directed toward an agent for decreasing the toxicity of a wide spectrum of chemotherapeutic agents.

Dihydrogen phosphorothioate compounds are known to be effective as antiradiation agents. See U.S. Patent No. 3,892,824 to Piper et al and Sweeney, A Survey of Compounds from the Antiradiation Drug Development Program of the U.S. Army Medical Research and Development Command, published by the Walter Reed Army Institute of Research, Washington D.C. (1979).

Int. J. Rad., Onc., Biol. Phys., 10(9) (1984), 1561-1564 describes that WR-2721 can protect normal cells against the cytotoxic effects of the anticancer nitrogen mustard (HN2), while potentiating its cytotoxicity against AKR leukaemia.

Cancer Clin. Trials, 4, (1981), 12, 14 discloses the ability of WR-2721 to protect bone marrow colony forming units against the cell kill of various cytotoxic chemotherapeutic agents, including nitrogen mustard, cyclophosphamide, BCNU, cis-platinum and 5-fluorouracil.

The present invention concerns the use of S-3-(3-methylaminopropylamino)propyl dihydrogen phosphorothioate for obtaining a pharmaceutical composition to decrease the toxicity of chemical therapeutic agents administered in cancer chemotherapy.

By chemical therapeutic agents, there is contemplated the chemicals or compositions administered to cancer patients during the course of the patient's chemotherapy. Exemplary of such chemotherapeutic agents are alkylating agents such as cyclophosphamide, melphalan and nitrogen mustard, as well as platinum agents such as carbaplatin and cisplatin.

By oral administration, there is contemplated the preparation of S-3-(3-methylaminopropylamino)propyl dihydrogen phosphorothioate in any dosage form capable of oral administration. Such dosage forms include tablets, capsules, caplets, solutions and the like.

The oral dosage forms of the present invention may contain pharmaceutically acceptable inert ingredients. As such inert ingredients there are contemplated pharmaceuticals, carriers, excipients, fillers, etc. which do not interfere with the activity of the compound.

Also, fillers such as clays or siliceous earth may be utilized if desired to adjust the size of the dosage form.

Further ingredients such as excipients and carriers may be necessary to impart the desired physical properties of the dosage form. Such physical properties are, for example, release rate, texture and size of the dosage form. Examples of excipients and carriers useful in oral dosage forms are waxes such as beeswax, castor wax glycowax and carnauba wax, cellulose compounds such as methylcellulose, ethylcellulose, carboxymethylcellulose, cellulose acetate phthalate, hydroxypropylcellulose and hydroxypropylmethylcellulose, polyvinyl chloride, polyvinyl pyrrolidone, stearyl alcohol, glycerin monostearate, methacrylate compounds such as polymethacrylate, methyl methacrylate and ethylene glycol dimethacrylate, polyethylene glycol and hydrophilic gums.

Also in accordance with the present invention, there is provided a liquid-based dosage form suitable for the administration of the composition to a patient. The liquid base for this dosage form may be any liquid capable of transporting the composition into the body of a patient without disrupting the activity of the compound or harm the patient. Exemplary of such a liquid is an isotonic solution. The isotonic solution may also contain conventional additives therein such as sugars. These solutions can be used in the preparation of oral, intravenous or inhalation composition.

Thus, the compositions according to the present invention may be admixed according to known procedures using known excipients.

As an effective amount of the compound of the present invention, there is contemplated any amount which would serve to decrease the toxicity of chemotherapeutic agents. For example, a dosage of between about 50 to about 2500 mg/m$^2$ body surface area of the patient is contemplated. A preferred dosage according to the present invention is from about 300 to about 1000 mg/m$^2$ body surface area of the patient. The active ingredient may be administered in single or divided doses.

S-3-(3-methylaminopropylamino)propyl dihydrogen phosphorothioate can be depicted as follows:

$CH_3$-NH-$(CH_2)_3$-NH-$(CH_2)_3$-S-$PO_3H_2$.

EP 0 391 971 B1

S-3-(3-methylaminopropylamino)propyl dihydrogen phosphorothioate may be prepared in accordance with the following procedure:

Preparation of N-Methyl-N,N'-trimethylenebis-p-toluenesulfonamide (1). - A freshly prepared solution of p-toluenesulfonyl chloride (90.8 g, 0.476 mole) in N,N-dimethylformamide (200 ml) is added during 45 min. with moderate external cooling to a stirred solution of N-methyl-1,3-propanediamine (41.9 g, 0.476 mole) in N,N-dimethylformamide (150 ml) at such a rate that the temperature does not exceed 40°. The mixture is stirred 45 min. longer at room temperature and then poured into cold water (1.2 l). The white gum that precipitated solidifies on standing. The crude product is collected, pulverized, and washed thoroughly with water. Recrystallization from ethanol affords the pure product, m.p. 93° (Kofler Heizbank), in 79% yield (74.4 g).

Anal. Calcd. for $C_{18}H_{24}N_2O_4S_2$: C, 54.52; H, 6.10; S, 16.17. Found: C, 54.33; H, 5.92; S, 16.4.

Preparation of 3-Chloropropyl Acetate (2). - Acetic anhydride (114 g, 1.12 mol) is added in a thin stream to a stirred mixture of 3-chloro-1-propanol (94.5 g, 1.00 mol) and glacial HOAc (50 ml). The solution is refluxed 2 h, cooled, and poured into $H_2O$ (200 ml). The layers are separated, and the aqueous layer is thoroughly extracted with $Et_2O$ (five times with 100-ml portions). The original organic layer is then combined with the $Et_2O$ solution, and the resultant solution is washed several times with $H_2O$ followed by saturated $NaHCO_3$ solution and finally with $H_2O$. The dried ($MgSO_4$) solution is fractionally distilled under reduced pressure to give 2, bp 63-66° (12-14 mm) [G.M. Bennett and F. Heathcoat, J. Chem. Soc., 268 (1929). bp 66° (14 mm)], in 80% yield (109 g).

Preparation of Trisodium phosphorothioate. - Thiophosphoryl chloride (56.5 g, 0.333 mole) is added to a solution of sodium hydroxide (80.0 g, 2.00 moles in 500 ml of water), and the mixture is heated with vigorous magnetic stirring to 83-84°. The heat source (Glas-Col mantle) is then immediately removed, and the mixture is quickly cooled to 75-77° by means of a water bath. When the water bath is removed, the temperature of the vigorously stirred mixture gradually rises spontaneously. The temperature is allowed to rise to 83-84°, and the mixture is again cooled rapidly back to 75-77°. This process of alternately cooling and allowing spontaneous temperature rise is repeated about six times, or until so little unreacted thiophosphoryl chloride remains that the spontaneous rise in temperature no longer occurs. The mixture, which is yellow in color, is then heated at 82-84° with continued stirring until the oily droplets of thiophosphoryl chloride disappear. [The total reaction period required is about 1 h. As short a reaction time as possible is desired.] Immediately after the mixture becomes clear, it is chilled rapidly in an ice-water bath to about 4°. The crystalline hydrated form of the product commences precipitating when the solution becomes cold. The mixture is then allowed to stand in the refrigerator at 4° for about 16 h. The crystalline precipitate is collected, pressed as dry as possible on the funnel, and washed with absolute ethanol (100 ml). The precipitate is then removed from the funnel and dissolved in water (250 ml) at 45°. The solution is filtered immediately. Absolute ethanol (200 ml) is gradually added with swirling to the filtrate, and the mixture is then cooled in a cold water bath to about 20°. The reprecipitated product is collected and washed with ethanol (100 ml). The product is then dehydrated by adding it to dry methanol (600 ml) and stirring the resultant mixture under anhydrous conditions for 1.5 h. The white methanol-insoluble solid is collected and dried for approximately 30 min at 100° in vacuo over phosphorus pentoxide. The anhydrous trisodium phosphorothioate thus obtained is a white powder amounting to about 50g (83% yield), and should be stored in a freezer under anhydrous conditions.

Preparation of N-(2-Acetoxypropyl)-N'-methyltrimethylenebis-p-toluenesulfonamide (3). A solution of (1) (39.5 g., 0.100 mole) in N,N-dimethylformamide (12.5 ml.) is added during 1 h to a stirred suspension of sodium hydride (4.00 g. of 60% oil dispersion, 0.100 mole of NaH) in N,N-dimethylformamide (75 ml) with moderate external cooling to maintain the temperature at about 30°. The mixture is stirred 1 h longer at room temperature, and a virtually clear solution results.

Freshly distilled (2) is added (13.5 g, 0.100 mole), and the resultant mixture is left to stir 42 h at room temperature. The mixture is then heated at 80-85° for 2 h. Most of the solvent is removed by distillation in vacuo, and the residual red-orange sirup is dissolved in benzene (250 ml). The benzene solution is washed with water (4 x 50 ml) and dried ($Na_2SO_4$). Removal of the benzene by evaporation under reduced pressure leaves an orange oil that is used as such.

Preparation of N-(3-Bromopropyl)-N'-methyl-1,3-propanediamine Dihydrobromide (4). - A stirred mixture of crude 3 described above (46.5 g) and 48% HBr (500 ml) is refluxed overnight and then slowly distilled through a 30-cm Vigreux column until 300 ml of distillate is collected during 8 hr. The solution that remained is cooled, treated with Norit®, filter (Celite®), and evaporated to dryness with aid of added portions of MeOH (aspirator, rotary evaporator, bath up to 70°). The residue is recrystallized successively from MeOH (Norit®)-$Et_2O$ and MeOH to give pure 4, mp 220-222° dec, in 40% yield (13.8 g), Anal. Calcd

4

EP 0 391 971 B1

for $C_7H_{19}BrN_2 \cdot 2HBR$: C, 22.66; H, 5.16; Br, 64.62; N, 7.55. Found: C, 22.69; H, 5.22; Br, 64.48; N, 7.68.

Preparation of S-3-(3-Methylaminopropylamino)propyl Dihydrogen Phosphorothioate (5) Trihydrate. - Solid (4) (7.80 g, 21.0 mmol) is added in portions to a stirred partial solution of $Na_3SPO_3$ (3.60 g, 20.0 mmol) in $H_2O$ (20 ml). The mixture, which soon becomes clear, is stirred at 25-30° for 1.75 h, poured into DMF (80 ml), and refrigerated overnight. The precipitate is collected, dissolved in $H_2O$ (20 ml), and reprecipitated by addition of EtOH. The crystalline product is collected with the aid of EtOH, washed successively with EtOH followed by $Et_2O$, air dried, and then equilibrated at constant 50% relative humidity to give pure (5)•$3H_2O$, mp 115-120°, in 85% yield (5.04g). Anal. Calcd for $C_7H_{19}N_2O_3PS \cdot 3H_2O$: C, 28.37; H, 8.50; N, 9.45; P, 10.45; P, 10.45; S, 10.82. Found: C, 28.35; H, 8.32; N, 9.48; P, 10.57; S, 10.91.

Preparation of 3-(3-Methylaminopropylamino)propanethiol Dihydrochloride (6). - The preparation of (5) described above is repeated (21.6 mmol of 4, 20.6 mmol of $Na_3SPO_3$),and the reprecipitated product (from $H_2O$-EtOH) is used for conversion to (6) without further characterization. The sample is dissolved in 3 N HCl (30 ml), and the solution is heated in a boiling $H_2O$ bath for 10 min. The cooled solution is diluted with EtOH (300 ml), and $Et_2O$ (200 ml) is added. The cloudy mixture is refrigerated overnight while crystalline solid separates. This material, collected under $N_2$ and suction dried under $N_2$ pressure is dissolved in MeOH (100 ml), and EtOH (500 ml) is added followed by a solution of dry HCl in EtOH (3 N, 25 ml). Crystalline (6), which separates readily, is collected under $N_2$, washed with EtOH followed by $Et_2O$, and dried in vacuo (25-30°, $P_2O_5$); the overall yield was 58% (2.80 g), mp 244-246° dec. Anal. Calcd for $C_7H_{18}N_2S \cdot 2HCl$: C, 35.74; H, 8.57; N, 11.91; S, 13.63; SH, 14.06. Found: C, 35.59; H, 8.69; N, 11.86; S, 13.44; SH, 14.28.

Illustrative examples of the present invention follow.

EXAMPLE I

1000 mg of S-3-(3-methylaminopropylamino)propyl dihydrogen phosphorothioate is suspended in an isotonic solution. 200 mg/m² body surface area of S-3-(3-methylaminopropylamino)propyl dihydrogen phosphorothioate thus suspended is administered to a patient undergoing chemotherapy with cisplatin.

EXAMPLE II

500 mg of S-3-(3-methylaminopropylamino)propyl dihydrogen phosphorothioate is suspended in an isotonic solution. 500 mg/m² body surface area of S-3-(3-methylaminopropylamino)propyl dihydrogen phosphorothioate thus suspended is administered to a patient undergoing chemotherapy with nitrogen mustard.

EXAMPLE III

1000 mg of S-3-(3-methylaminopropylamino)propyl dihydrogen phosphorothioate is admixed with hydroxypropylcellulose and stearyl alcohol. The mixture is then compressed into tablet form. 200 mg/m² body surface area of S-3-(3-methylaminopropylamino)propyl dihydrogen phosphorothioate thus prepared is administered to a patient undergoing chemotherapy with cyclophosphamide, N,N-bis(2-chloroethyl)-tetrahydro-2H-1,3,2-oxazaphosphorin-2-amine.

EXAMPLE IV

700 mg of S-3-(3-methylaminopropylamino)propyl dihydrogen phosphorothioate is admixed with hydroxypropylcellulose and glycowax. The mixture is then compressed into tablet form. 500 mg/m² body surface area of S-3-(3-methylaminopropylamino)propyl dihydrogen phosphorothioate thus prepared is administered to a patient undergoing chemotherapy with melphalan, 4-[bis(2-chloroethyl)amino]-L-phenylalanine.

5

| Murine Toxicity Studies with S-3-(3-methylaminopropylamino)propyl dihydrogen phosphorothioate trihydrate | | |
|---|---|---|
| Drug Treatment (single dose) | Day 4 WBC Nadir: % Control[§] | Lethal Toxicity |
| cisplatin, 17 mg/kg i.v. S-3-(3-methylaminopropylamino)propyl dihydrogen phosphorothioate trihydrate, 750 mg/kg i.p. + cisplatin, 17 mg/kg i.v.* | 33% 84% | 80% None |
| cisplatin, 12 mg/kg i.v. S-3-(3-methylaminopropylamino)propyl dihydrogen phosphorothioate trihydrate, 1000 mg/kg p.o. + cisplatin, 12 mg/kg iv.* | 52% 82% | 15% None |

*25 minutes after S-3-(3-methylaminopropylamino)propyl dihydrogen phosphorothioate trihydrate
[§]5 male $CD2F_1$ mice per group
S-3-(3-methylaminopropyl-amino)propyl dihydrogen phosphorothioate trihydrate(100 mg/ml) was dissolved at 4 degrees C in Lactated Ringer's and 5% Dextrose, pH adjusted to 7.2-7.3 with sodium bicarbonate, immediately prior to use
cisplatin was dissolved in 0.85% sodium chloride at 1.2 mg/ml

Evaluation of the Effect of S-3-(3-methylaminopropylamino)propyl dihydrogen phosphorothioate trihydrate on the Lethality of Cisplatin

Nontumored Charles River, Portage C2F1 Female Animals
Treatment: Day 1 Only

| Name | Dosage (mg/kg/dose) | Route & Schedule | Day of Death (Mean Animal Weight in Grams) | | | | | | | | | | | | | 30-Day Surv/Tot | LD10 (mg/kg/dose) |
|---|---|---|---|---|---|---|---|---|---|---|---|---|---|---|---|---|---|
| | | | 1 | 2 | 3 | 4 | 5 | 6 | 7 | 8 | 9 | 15 | 16 | 21 | 28 | | |
| Cisplatin | 39 | IP | (19) | | 1(16) | | 1 | 1 | 1(14) | | 1 | (20) | | (21) | (22) | 2/6 | 11 |
| | 28 | | (20) | | (17)1 | 1 | | | 3(13) | | | (19) | | (21) | (22) | 1/6 | |
| | 20 | | (19) | | (16) | | | 2(15) | | | | (19) | | (20) | (22) | 4/6 | |
| | 14 | | (19) | | (17) | | | | (17)1 | 1 | | (20) | | (21) | (22) | 5/6 | |
| S-3-(3-methylaminopropylamino)propyl dihydrogen phosphorothioate trihydrate | 1000 | PO | (19) | | (19) | | | 4 | (19) | | | (20) | | (21) | (21) | 6/6 | >1000 |
| 1) Cisplatin  2) S-3-(3-methylaminopropylamino)propyl dihydrogen phosphorothioate trihydrate | 1) 39  2) 1000 | 1) IP  2) PO, 30 min before cisplatin | (19) | 1 | 1(17) | | | 4 | | | | (19) | | (20) | (22) | 0/6 | 18 (Cisplatin) |
| | 1) 28  2) 1000 | | (19) | 1 | (18) | | | | 1(16) | | | (19) | | (20) | (22) | 4/6 | |
| | 1) 20  2) 1000 | | (20) | 1 | (18) | | | | 1(16) | | | (20) | | (20) | (22) | 4/6 | |
| | 1) 14  2) 1000 | | (20) | | (18) | | | | (16) | | | (20) | | (20) | (22) | 6/6 | |

Cisplatin prepared in saline (soluble).
S-3-(3-methylaminopropylamino)propyl dihydrogen phosphorothioate trihydrate prepared in 5% dextrose lactate in Ringer's solution buffered with sodium bicarbonate to pH 7.2-7.4 (soluble).

## Claims

1. Use of S-3-(3-methylaminopropylamino)propyl dihydrogen phosphorothioate for obtaining a pharmaceutical composition to decrease the toxicity of chemical therapeutic agents administered in cancer chemotherapy.

7

2. Use according to Claim 1, wherein said S-3-(3-methylaminopropylamino)propyl dihydrogen phosphorothioate is administered in an amount not greater than 2500 mg/m$^2$ body surface area of said patient.

3. Use according to Claim 1, wherein said S-3-(3-methylaminopropylamino)propyl dihydrogen phosphorothioate is administered in an amount of beween about 300 and 1000 mg/m$^2$ body surface area of said patient.

4. Use according to any of the preceding claims, wherein the S-3-(3-methylaminopropylamino)propyl dihydrogen phosphorothioate is administered in an oral dosage form.

## Patentansprüche

1. Verwendung von S-3-(3-Methylaminopropylamino)propyl-dihydrogen-phosphorthioat zur Herstellung einer pharmazeutischen Zusammensetzung zur Verminderung der Toxizität von chemischen Therapeutika, die bei der Chemotherapie zur Krebsbekämpfung verabreicht werden.

2. Verwendung nach Anspruch 1, wobei das S-3-(3-Methylaminopropylamino)propyl-dihydrogen-phosphorthioat in einer Menge von höchstens 2500 mg/m$^2$ Körperoberfläche des Patienten verabreicht wird.

3. Verwendung nach Anspruch 1, wobei das S-3-(3-Methylaminopropylamino)propyl-dihydrogen-phosphorthioat in einer Menge zwischen ca. 300 und 1000 mg/m$^2$ Körperoberfläche des Patienten verabreicht wird.

4. Verwendung nach einem der vorangegangenen Ansprüche, wobei das S-3-(3-methylaminopropylamino)propyl-dihydrogen-phosphorthioat in einer oralen Dosierungsform verabreicht wird.

## Revendications

1. Utilisation de dihydrogénophosphorothioate de S-3-(3-méthylaminopropylamino)propyle pour obtenir une composition pharmaceutique destinée à diminuer la toxicité d'agents thérapeutiques chimiques administrés en chimiothérapie cancéreuse.

2. Utilisation suivant la revendication 1, dans laquelle ledit dihydrogénophosphorothioate de S-3-(3-méthylaminopropylamino)propyle est administré selon une quantité non supérieure à 2500 mg/m$^2$ de surface corporelle du patient.

3. Utilisation suivant la revendication 1, dans laquelle ledit dihydrogénophosphorothioate de S-3-(3-méthylaminopropylamino)propyle est administré selon une quantité comprise entre 300 et 1000 mg/m$^2$ de surface corporelle du patient.

4. Utilisation suivant une des revendications précédantes, le dihydrogénophosphorothioate de S-3-(3-méthylaminopropylamino)propyle étant administré sous form de dosage orale.